# EUROPEAN PATENT SPECIFICATION

(11) **EP 4 388 068 B1**
(45) Date of publication and mention of the grant of the patent: **10.09.2025**
(21) Application number: 22789991.1
(22) Date of filing: 04.10.2022
(51) Int. Cl.: C11D 7/02, C11D 11/00

(54) **BIOFILM REMOVAL**
BIOFILMENTFERNUNG
ÉLIMINATION DE BIOFILM

(30) Priority: 13.10.2021 GB 202114645
(43) Date of publication of application: 26.06.2024
(73) Proprietor: Partners for Endoscopy Limited, Stoke on Trent ST4 3PE (GB)
(72) Inventor: HARTLEY, Robert John, Fenton Staffordshire ST4 3PE (GB); DUONG, Thien Duc, Fenton Staffordshire ST4 3PE (GB); SUTHERLAND, Andrew James, Birmingham B4 7ET (GB); WORTHINGTON, Tony, Birmingham B4 7ET (GB)
(74) Representative: Swindell & Pearson Limited
(86) International application number: PCT/GB2022/052505
(87) International publication number: WO 2023/062337

(56) References cited:
- WO-A1-2015/002932
- US-A- 6 004 510

## Description

### TECHNOLOGICAL FIELD

Embodiments of the present disclosure relate to a mixture for removing biofilm from surfaces. Some relate to a mixture for removing biofilm from the biopsy channel of an endoscope.

### BACKGROUND

Biofilms include a number of microorganisms within an extracellular matrix, and can form on a wide variety of surfaces. For instance, mature biofilms can form on the inner walls of a biopsy channel of an endoscope.

Biofilms can contain a wide spectrum of pathogenic microorganisms including gram-positive bacteria, gram-negative bacteria, and fungi. Bacteria within a biofilm can be 10 to 1000 times more difficult to remove than planktonic bacteria. Due to the risk of infection, it is highly desirable to remove biofilms from surfaces, especially in environments such as hospitals or food preparation areas. However, present means of removing biofilm can be slow, damage the surface, and/or inadequately remove the whole spectrum of pathogenic microorganisms from the surface. Methods for treating a surface characterized by microbial infection or colonization using hypochlorous acid and a silver additive are known from WO2015/002932A1. US6004510A dicloses a process for the disinfection of a surface with a hygiene agent using hydrogen peroxide and transition metal catalysts, such as manganese.

### BRIEF SUMMARY

The term "at least" used in this specification is defined as greater than or equal to (i.e., ≥). The term "up to" used in this specification is defined as a smaller value than or equal to (i.e., ≤).

According to various, but not necessarily all, embodiments there is provided a cleaning mixture for removing biofilm from surfaces, the mixture comprising: silver particles and/or particles surface modified with silver; manganese dioxide particles and/or particles surface modified with manganese oxide; and water.

The mixture may further comprise hydrogen peroxide. Possibly, the mixture comprises up to 5 wt.% hydrogen peroxide. Possibly, the mixture comprises up to 2 wt.% hydrogen peroxide.

The mixture may comprise silver particles. Possibly, the silver particles are present in the mixture at a concentration of from 0.1 mg/mL to 1 mg/mL. Possibly, the silver particles are present in the mixture at a concentration of from 0.2 mg/mL to 0.5 mg/mL.

Possibly, the silver particles have a hydrodynamic diameter of up to 1 µm. Possibly, the silver particles have a hydrodynamic diameter of up to 500 nm. Possibly, the silver particles have a hydrodynamic diameter of up to 200 nm.

The mixture may comprise manganese dioxide particles. Possibly, the manganese dioxide particles are present in the mixture at a concentration of from 0.0002 mg/mL to 0.005 mg/mL. Possibly, the manganese dioxide particles are present in the mixture at a concentration of from 0.0005 mg/mL to 0.003 mg/mL.

Possibly, the manganese dioxide particles have a hydrodynamic diameter of up to 1 µm. Possibly, the manganese dioxide particles have a hydrodynamic diameter of up to 500 nm.

The mixture may further comprise a capping agent. The capping agent may be a water-soluble polymer capping agent. The polymer capping agent may be polyvinylpyrrolidone.

The mixture may comprise silver particles and manganese dioxide particles.

The mixture may comprise particles surface modified with silver and particles surface modified with manganese oxide. Possibly, at least some of the particles surface modified with silver and at least some of the particles surface modified with manganese oxide are particles surface modified with both silver and manganese oxide. The particles surface modified with both silver and manganese oxide may be zeolite particles surface modified with both silver and manganese oxide.

According to various, but not necessarily all, embodiments there is provided a cleaning mixture for removing biofilm from surfaces, the mixture consisting of: silver particles and/or particles surface modified with silver; manganese dioxide particles and/or particles surface modified with manganese oxide; and water.

The mixture may further consist of hydrogen peroxide.

According to various, but not necessarily all, embodiments there is provided a cleaning mixture for removing biofilm from surfaces, the mixture comprising: silver containing particles; manganese oxide containing particles; and water.

The surface of the silver containing particles may comprise silver. The surface of the manganese oxide containing particles may comprise manganese oxide.

The mixture may further comprise hydrogen peroxide. Possibly, the mixture comprises up to 5 wt.% hydrogen peroxide. Possibly, the mixture comprises up to 2 wt.% hydrogen peroxide.

According to various, but not necessarily all, embodiments there is provided a method of removing biofilm from a surface, the method comprising: applying the cleaning mixture of any of the preceding paragraphs to the surface.

Hydrogen peroxide may be added to the mixture up to 5 minutes before applying the cleaning mixture to the surface.

The cleaning mixture may be applied to the surface for up to 15 minutes.

The surface may be the inner surface of a tube. The cleaning mixture may be applied by passing the mixture through the tube. The tube may be the biopsy channel of an endoscope.

The method may further comprise applying an enzymatic solution to the surface. The method may comprise passing the enzymatic solution through the biopsy channel prior to passing the cleaning mixture through the biopsy channel.

The method may further comprise rinsing the surface with water after the application of the cleaning mixture to the surface.

According to various, but not necessarily all, embodiments there is provided a system for removing biofilm from the biopsy channel of an endoscope, the system comprising: a reservoir containing the cleaning mixture of any of the preceding paragraphs; and an inlet, which is arranged to direct fluid into an internal channel of the endoscope.

The system may further include a bath for locating the endoscope. The system may include one or more inlets into the bath. The system may include a drain for draining liquid from the bath.

Examples of the disclosure also provide a method of producing manganese dioxide particles, comprising: mixing a manganese (II) salt and an acetate salt with a capping agent in water to form a first solution; and adding an oxidising agent to the first solution.

The method may be carried out at room temperature.

The capping agent may be a water-soluble polymer capping agent. The polymer capping agent may be polyvinylpyrrolidone.

The manganese (II) salt may be manganese acetate. The acetate salt may be manganese acetate. The oxidising agent may be potassium permanganate. The ratio of the moles of manganese acetate in the first solution to the moles of potassium permanganate added to the first solution may be from 1:1 (mol Mn(CH₃COO)₂:mol KMnO4) to 2:1 (mol Mn(CH₃COO)₂:mol KMnO4).

Tannic acid may also be mixed with the manganese acetate and capping agent in water, prior to the addition of the potassium permanganate.

The solution may be stirred prior to and/or after the addition of the potassium permanganate. The potassium permanganate may be added in a dropwise fashion.

According to various, but not necessarily all, embodiments there is provided examples as claimed in the appended claims.

### BRIEF DESCRIPTION

Some examples will now be described with reference to the accompanying drawings in which:
Fig. 1 shows a graph illustrating the amount of *Pseudomonas aeruginosa* bacteria remaining on a biopsy channel of an endoscope after treatment of the channel with hydrogen peroxide, after treatment of the channel with a mixture of zeolite and hydrogen peroxide, after a control treatment is applied to the channel, and after treatment of the channel with a zeolite;
Fig. 2 shows a graph illustrating the amount of *Enterococcus hirae* bacteria remaining on a biopsy channel of an endoscope after a control treatment is applied to the channel, after treatment of the channel with a mixture of silver modified zeolite and hydrogen peroxide, after treatment of the channel with a mixture of manganese oxide and silver modified zeolite, and after treatment of the channel with a mixture of manganese and silver modified zeolite and hydrogen peroxide;
Fig. 3 shows a graph illustrating the amount of various pathogens remaining on an untreated surface, and the amount of various pathogens remaining on a surface treated with an example cleaning mixture;
Figs. 4A - 4F show a system and method for applying an enzymatic solution to an endoscope;
Figs. 5A - 5D show a system and method for rinsing an endoscope with water; and
Figs. 6A - 6D show a system and method for applying a cleaning mixture to an endoscope; and
Fig. 7 shows an example method for removing biofilm from an endoscope.

### DETAILED DESCRIPTION

### Cleaning mixture

Examples of the disclosure provide a cleaning mixture for removing biofilm from surfaces, the mixture comprising:
silver particles and/or particles surface modified with silver;
manganese dioxide particles and/or or particles surface modified with manganese oxide; and
water.

### First example cleaning mixture

In a first example of the cleaning mixture for removing biofilm from surfaces, the mixture comprises silver particles, manganese dioxide particles, and water.

The first example cleaning mixture for removing biofilm from surfaces disclosed herein includes silver particles. In some examples, the silver particles have a hydrodynamic diameter of up to 100 µm, as measured using dynamic light scattering. The hydrodynamic diameter is the diameter of a hypothetical hard sphere that diffuses with the same speed as the particle being measured. The hydrodynamic diameters described herein are as measured using dynamic light scattering.

In some examples, the silver particles have a hydrodynamic diameter of up to 50 µm, or up to 20 µm. Preferably, the silver particles have a hydrodynamic diameter of up to 1 µm, up to 500 nm, up to 200 nm, or up to 150 nm. Particles with smaller sizes have been found to be more effective for use in a cleaning solution, as the particles can remain dispersed for longer time periods. The silver particles may have a hydrodynamic diameter of 10 nm to 200 nm. Most preferably, the silver particles have a hydrodynamic diameter of 50 nm to 150 nm (such as 127 nm).

The cleaning mixture for removing biofilm from surfaces disclosed herein further includes manganese dioxide particles. In some examples, the manganese dioxide particles have a hydrodynamic diameter of up to 100 µm, as measured using dynamic light scattering. The hydrodynamic diameter is the diameter of a hypothetical hard sphere that diffuses with the same speed as the particle being measured. The hydrodynamic diameters described herein are as measured using dynamic light scattering.

In some examples, the manganese dioxide particles have a hydrodynamic diameter of up to 50 µm, or up to 20 µm. Preferably, the manganese dioxide particles have a hydrodynamic diameter of up to 1 µm, up to 500 nm, or up to 300 nm. Particles with smaller sizes have been found to be more effective for use in a cleaning solution, as the particles can remain dispersed for longer time periods. The manganese dioxide particles may have a hydrodynamic diameter of 10 nm to 300 nm. Most preferably, the manganese dioxide particles have a hydrodynamic diameter of 150 nm to 250 nm (such as 206 nm).

In some examples, the silver particles are present in the mixture at a concentration of at least 0.01 mg/mL, at least 0.05 mg/mL, at least 0.1 mg/mL, at least 0.2 mg/mL, or at least 0.3 mg/mL. In some examples, the silver particles are present in the mixture at a concentration of up to 10 mg/mL, up to 5 mg/mL, up to 1 mg/mL up to 0.7 mg/mL, up to 0.5 mg/mL, or up to 0.4 mg/mL. Preferably, the silver particles are present in the mixture at a concentration of from 0.1 mg/mL to 1 mg/mL. Most preferably, the silver particles are present in the mixture at a concentration of from 0.2 mg/mL to 0.5 mg/mL, such as 0.326 mg/mL.

In some examples, the manganese dioxide particles are present in the mixture at a concentration of at least 0.0001 mg/mL, at least 0.0003 mg/mL, at least 0.0005 mg/mL, or at least 0.0008 mg/mL. In some examples, the manganese dioxide particles are present in the mixture at a concentration of up to 1 mg/mL, up to 0.1mg/mL, up to 0.01 mg/mL, up to 0.007 mg/mL, up to 0.005 mg/mL, up to 0.003 mg/mL, or up to 0.002 mg/mL. Preferably, the manganese dioxide particles are present in the mixture at a concentration of from 0.0002 mg/mL to 0.005 mg/mL. Most preferably, the manganese dioxide particles are present in the mixture at a concentration of from 0.0005mg/mL to 0.003 mg/mL, such as 0.001 mg/mL.

In some examples, the mixture further comprises a capping agent. Preferably, the capping agent comprises at least one of: polyethylene glycol, ethylenediaminetetraacetic acid, polyvinyl pyrrolidone, polyvinyl alcohol, polyallylamine hydrochloride, dodecanthiol, or chitosan. Most preferably, the capping agent is a water-soluble polymer capping agent, such as polyvinyl pyrrolidone, polyethylene glycol, ethylenediaminetetraacetic acid, polyvinyl alcohol, or polyallylamine hydrochloride. The capping agent may be polyvinyl pyrrolidone.

In some examples, the manganese dioxide particles and/or the silver particles are dispersed in the water, such that the cleaning mixture is considered a dispersion.

The water in the cleaning mixture could for instance be deionised water, distilled water or tap water. The mixture could include additional components dissolved within the water, such as for instance sodium chloride in a saline solution. The saline solution may be phosphate buffered saline.

Preferably, the mixture further comprises hydrogen peroxide. In some examples, the mixture comprises up to 5 wt.% hydrogen peroxide, up to 3 wt.% hydrogen peroxide, up to 2 wt. % hydrogen peroxide, or up to 1.5 wt.% hydrogen peroxide. In some examples, the mixture comprises at least 0.1. wt.% hydrogen peroxide, at least 0.3 wt.% hydrogen peroxide or at least 0.5 wt.% hydrogen peroxide. Preferably, the mixture comprises 0.3 wt.% to 3 wt.% hydrogen peroxide. Most preferably, the mixture comprises 0.5 wt.% to 1.5 wt.% hydrogen peroxide, such as 1 wt.% hydrogen peroxide.

In some examples, the hydrogen peroxide is added to the mixture up to 15 minutes before the application of the mixture to the surface. Preferably, the hydrogen peroxide is added to the mixture up to 5 minutes before the application of the mixture to the surface with biofilm.

### Preparation of the first example cleaning mixture

The first example cleaning mixture is prepared by a method comprising: preparing silver particles; preparing manganese dioxide particles; and mixing the silver particles and manganese dioxide particles in water.

Examples of the disclosure provide a method of producing manganese dioxide particles, comprising:
mixing a manganese (II) salt and an acetate salt with a capping agent in water to form a first solution; and
adding an oxidising agent to the first solution.

In some examples, the manganese (II) salt is manganese (II) acetate, manganese (II) sulphate, manganese (II) chloride, or manganese (II) nitrate. Preferably, the manganese (II) salt is manganese acetate.

In some examples, the acetate salt is manganese acetate or sodium acetate. Preferably, both the manganese (II) and acetate salts are manganese acetate.

Advantageously, it has been found that this method of forming manganese dioxide particles provides a well dispersed mixture, with a short reaction time. Furthermore, no heat source is required to perform the reaction. The method may be carried out at room temperature.

In some examples, the capping agent comprises at least one of: polyethylene glycol, ethylenediaminetetraacetic acid, polyvinyl pyrrolidone, polyvinyl alcohol, polyallylamine hydrochloride, dodecanthiol, or chitosan. Preferably, the capping agent is a water-soluble polymer capping agent, such as polyvinyl pyrrolidone, polyethylene glycol, ethylenediaminetetraacetic acid, polyvinyl alcohol, or polyallylamine hydrochloride. Most preferably, the capping agent is polyvinyl pyrrolidone.

In some examples, the oxidising agent includes at least one of: potassium permanganate, ammonium persulfate or sodium chlorate. Preferably, the oxidising agent is potassium permanganate.

Where the oxidising agent is potassium permanganate and the manganese (II) and acetate salts are manganese acetate, the ratio of the moles of manganese acetate in the first solution to the moles of potassium permanganate added to the solution may be from 1:1 (mol Mn(CH₃COO)₂:mol KMnO₄) to 2:1 (mol Mn(CH₃COO)₂:mol KMnO₄). Preferably, the ratio of the moles of manganese acetate in the first solution to the moles of potassium permanganate added to the solution is 3:2 (mol Mn(CH₃COO)₂:mol KMnO₄).

In some examples, the water is deionised water or distilled water.

In some examples, tannic acid is also mixed into the first solution prior to the addition of the oxidising agent.

In some examples, the solution is stirred prior to and/or after the addition of the oxidising agent. The oxidising agent may be added in a dropwise fashion.

An example method for producing manganese dioxide particles includes adding aqueous polyvinyl pyrrolidone solution (1 mL, 1 %w/v), aqueous manganese acetate solution (0.45 mL, 0.01 M), and aqueous tannic acid solution (1 mL, 0.001M) to deionised water (7 mL) under continuous stirring at 500 rpm in a 25 mL conical flask. To this solution, an aqueous solution of potassium permanganate (0.6 mL, 0.005 M) is added slowly in a dropwise fashion. After addition is complete, the mixture was left stirring for 15 minutes at room temperature prior to dilution in water (1:4 mixture:water), to provide a manganese dioxide particle stock mixture. The aqueous manganese acetate solution may be prepared by dissolving solid manganese acetate in deionised water. The aqueous potassium permanganate solution may be prepared by dissolving solid potassium permanganate in deionised water.

A comparative example method for producing manganese dioxide particles includes dissolving KMnO₄ (3 g) in deionised water (800 mL), followed by the addition of ethyl acetate (120 mL). The reaction mixture is stirred continuously at 50 - 60 °C overnight. After reaction, the mixture is filtered and the manganese dioxide cake is washed with water and then ethanol. The sample is then dried in the oven overnight at 60°C. This comparative example method can produce manganese dioxide particles for use in the cleaning mixture, but is much slower than the example method described previously, and also requires a heat source.

An example silver particle synthesis includes adding aqueous silver nitrate solution (1 mL, 0.1 M) and aqueous polyvinylpyrrolidone solution (1 mL, 1% w/v) to deionised water (7 mL) under continuous stirring at 500 rpm in a 25 mL conical flask. To this solution, a reducing agent such as a freshly made-up aqueous solution of sodium borohydride (1 mL, 0.015 M) is added slowly in a dropwise fashion. After addition was complete, the mixture was left stirring for 15 minutes at room temperature, to form a silver particle stock mixture. Tollens' reagent [Ag(NH₃)₂]⁺ could be used in place of silver nitrate in other examples. An alternative reducing agent to sodium borohydride could be for instance sodium citrate, sodium ascorbate, lithium aluminium hydride, diisobutylaluminum hydride, or polyethylene glycol (PEG). The aqueous silver nitrate solution may be prepared by dissolving solid silver nitrate in deionised water.

To form the cleaning mixture for removing biofilm from surfaces, a silver particle mixture may be mixed with a manganese dioxide particle mixture. The silver particle mixture and the manganese dioxide particle mixture can be formed using any suitable method, such as those described above. As an example, 1 mL of the silver particle stock mixture described previously is mixed with 0.5 mL of the manganese dioxide particle stock mixture described previously, to provide a first stock cleaning mixture for removing biofilm from surfaces.

Preferably, the cleaning mixture for removing biofilm from surfaces is mixed with hydrogen peroxide prior to use. For instance, 1.5 mL of the first stock cleaning mixture could be diluted with 1 mL of deionised water, then 0.5 mL of aqueous 6 wt.% hydrogen peroxide solution could be added to the mixture.

### Second example cleaning mixture

A second example cleaning mixture for removing biofilm from surfaces comprises particles surface modified with silver, particles surface modified with manganese oxide, and water.

Preferably, the particles surface modified with silver and the particles surface modified with manganese oxide comprise particles surface modified with both silver and manganese oxide. In other words, at least some of the particles include both manganese oxide and silver on their outer surface. The particles surface modified with both manganese oxide and silver may be provided in the water at a concentration of 0.5 mg/mL of water to 1.5 mg/mL of water, such as 1 mg of surface modified particles per mL of water.

In some examples, the particles surface modified with silver and the particles surface modified with manganese oxide are zeolite particles surface modified with both manganese oxide and silver.

In some examples, the particles surface modified with both silver and manganese oxide have a hydrodynamic diameter of up to 100 µm, as measured using dynamic light scattering. The hydrodynamic diameter is the diameter of a hypothetical hard sphere that diffuses with the same speed as the particle being measured. The hydrodynamic diameters described herein are as measured using dynamic light scattering.

In some examples, the particles surface modified with both silver and manganese oxide have a hydrodynamic diameter of up to 70 µm, or up to 50 µm. The particles surface modified with both silver and manganese oxide may have a hydrodynamic diameter of 20 µm to 100 µm, such as 50 µm to 70 µm.

In some examples, the loading of manganese on surface of the particles is between 0.5 wt.% and 10 wt. %. This wt.% loading is the total weight of manganese (i.e., manganese/manganese ions, not manganese oxide) added to the particles, calculated as a percentage of the total weight of the particles before surface modification. Preferably, the loading of manganese on the particles is between 1 wt.% and 5 wt. %. Most preferably, the loading of manganese on the particles is between 2 wt.% and 4 wt. %, such as 3 wt.%.

In some examples, the loading of silver on the surface of the particles is between 20 wt.% and 80 wt. %. This wt.% loading is the total weight of silver (i.e., silver/silver ions, not silver oxide) added to the particles, calculated as a percentage of the total weight of the particles before surface modification. Preferably, the loading of silver on the particles is between 30 wt.% and 60 wt. %. Most preferably, the loading of silver on the particles is between 35 wt.% and 50 wt. %, such as 43 wt.%.

In some examples, the particles surface modified with silver and the particles surface modified with manganese oxide are dispersed in the water, such that the cleaning mixture is considered a dispersion.

The water in the cleaning mixture could for instance be deionised water, distilled water or tap water. The mixture could include additional components dissolved within the water, such as for instance sodium chloride in a saline solution. The saline solution may be phosphate buffered saline.

Preferably, the mixture further comprises hydrogen peroxide. In some examples, the mixture comprises up to 5 wt.% hydrogen peroxide, up to 3 wt.% hydrogen peroxide, up to 2 wt. % hydrogen peroxide, or up to 1.5 wt.% hydrogen peroxide. In some examples, the mixture comprises at least 0.1. wt.% hydrogen peroxide, at least 0.3 wt.% hydrogen peroxide or at least 0.5 wt.% hydrogen peroxide. Preferably, the mixture comprises 0.3 wt.% to 3 wt.% hydrogen peroxide. Most preferably, the mixture comprises 0.5 wt.% to 1.5 wt.% hydrogen peroxide, such as 1 wt.% hydrogen peroxide.

In some examples, the hydrogen peroxide is added to the mixture up to 15 minutes before the application of the mixture to the surface. Preferably, the hydrogen peroxide is added to the mixture up to 5 minutes before the application of the mixture to the surface with biofilm.

### Preparation of the second example cleaning mixture

The second example cleaning mixture is prepared by a method comprising: surface modifying particles with silver; surface modifying particles with manganese oxide; and mixing the surface modified particles in water. Preferably, the same particles are surface modified with both silver and manganese oxide. Most preferably, the particles are zeolite particles, which are surface modified with both silver and manganese dioxide.

An example synthesis of the manganese oxide surface modified particles and the silver surface modified zeolite particles includes dispersing 0.5 g of zeolite Y (Alfa Aesar Zeolite Y, sodium, product number 45862) in 100 mL of deionised water under stirring (500 rpm) for few minutes. To that mixture, 1 mL of 28 wt.% concentrated ammonium hydroxide, 0.5 mL of tetraethyl orthosilicate (TEOS) and 1 mL of (3-aminopropyl) triethoxysilane (APTES) were slowly added. This mixture was heated under stirring in a water bath at 50°C until dried, providing a white solid of amino-functionalised zeolite. After drying, a 10 mL of a 0.05 M aqueous solution of potassium permanganate, (KMnO₄) and 80 mL of a 0.1 M aqueous solution of silver nitrate, (AgNO₃) were mixed with the amino-functionalised zeolite. The mixture was then placed in an ultrasonic bath for 30 minutes at 45 kHz. The mixture is then dried in a water bath at 50 °C to provide the manganese oxide and silver surface modified zeolite particles.

### Synergistic effect

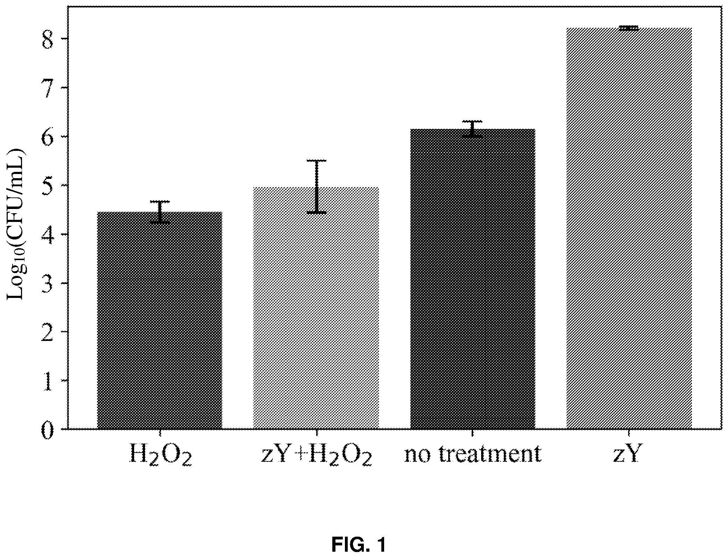

Figs. 1 to 3 show charts illustrating the synergistic effect provided by the ingredients of the cleaning mixtures described herein.

Fig. 1 is a chart illustrating the effectiveness of biofilm treatments applied to the biopsy channel of an endoscope. The biopsy channel of the endoscope harbours a biofilm containing *Pseudomonas aeruginosa.*

As a first step, to form the sample biopsy tubes harbouring the biofilm, the biopsy channel was first cut into small pieces, which are then submerged into Müller-Hilton broth. A single colony of *Pseudomonas aeruginosa* was added to the broth. At a second step, the pieces were kept in the broth at 37°C for 24 hours to allow the bacteria to grow. Also in the second step, the tube pieces were then washed 5 times with a copious amount of water to remove dangling bacteria, and to leave only fixed biofilm remaining on the surface of the tubes. The second step is then repeated three times to provide a mature biofilm.

To provide the data of Fig. 1, different treatment mixtures were applied for 5 minutes to the tubes with mature biofilm. The tubes with mature biofilm were prepared using the method above. After the treatment mixture is applied, the tubes are washed. The tubes are then placed in a centrifuge tube floating in an ultrasonic bath to release bacteria in the biofilm into solution. The number of colony forming units (CFU) is calculated via serial dilution and plate counting method. Each experiment is performed in triplicate.

From left to right along the chart of Fig. 1, the different treatment mixtures applied to the biopsy channel are an aqueous mixture including 1.5 wt.% hydrogen peroxide, an aqueous mixture including 1 mg/mL of zeolite Y and 1.5 wt.% hydrogen peroxide, a control treatment mixture including a phosphate-buffer saline, and an aqueous mixture including 1mg/mL of zeolite Y. As shown in the chart, hydrogen peroxide causes a reduction in the number of colony forming units of the *Pseudomonas aeruginosa* bacteria, but the unmodified zeolite Y alone does not cause a reduction. Thus zeolite Y alone did not provide appear to have a biocidal effect against the *Pseudomonas aeruginosa* bacteria.

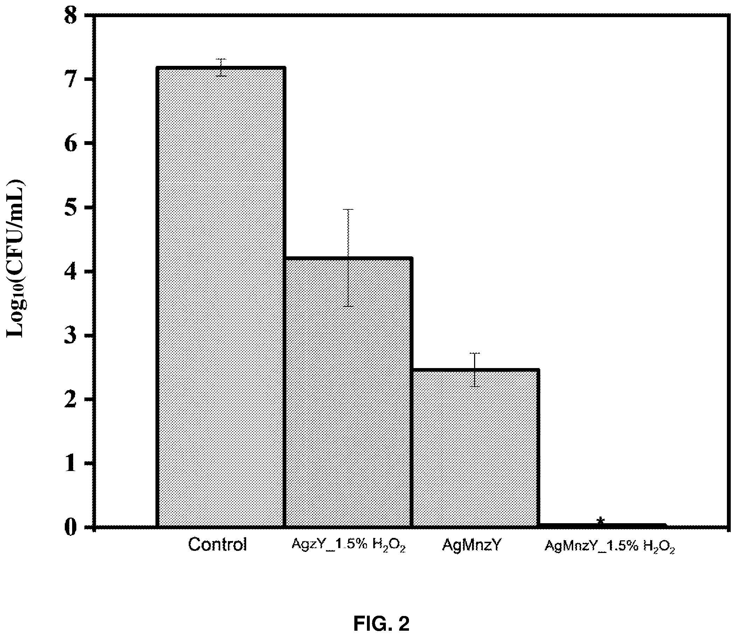

Fig. 2 is a chart illustrating the effectiveness of biofilm treatments when applied to the biopsy channel of an endoscope. The treatments are applied in the same manner as those of Fig. 1, except the second step of the preparation of the sample biopsy tubes was repeated only once, and also the biofilm contains *Enterococcus hirae,* rather than *Pseudomonas aeruginosa.* From left to right along the chart, the treatment mixtures applied to the biopsy channel are a control treatment including a phosphate-buffer saline, an aqueous mixture including 1mg/mL of silver surface modified zeolite Y and 1.5 wt.% hydrogen peroxide, a cleaning mixture including 1mg/mL of silver and manganese oxide surface modified zeolite Y, and a cleaning mixture including 1mg/mL of silver and manganese oxide surface modified zeolite Y and 1.5 wt.% hydrogen peroxide. As clearly illustrated in Fig. 2, silver and manganese oxide in combination provide a very strong biocidal effect, which is even further enhanced by the addition of hydrogen peroxide to the mixture. The silver modified particles alone produce a small biocidal effect, and testing has also shown that manganese oxide surface modified particles alone provide an insignificant biocidal effect, thereby illustrating that the strong biocidal effect of silver and manganese oxide in combination is synergistic.

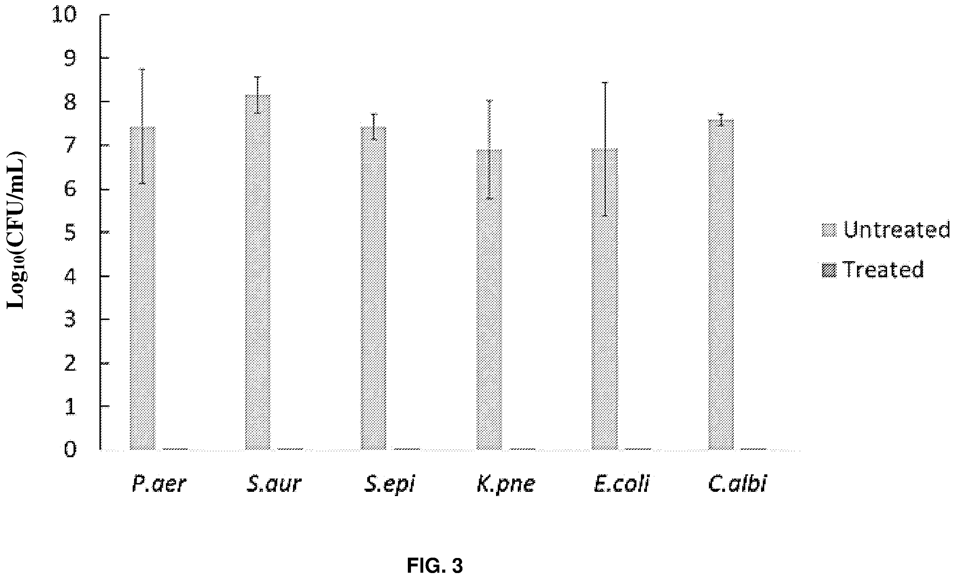

Fig. 3 is a chart illustrating the effectiveness of an example cleaning mixture when applied to a biofilm. The results of Fig. 3 were created using a similar method as described in relation to Figs. 1 and 2 above, except the treatment was tested against a number of different biofilms, with each biofilm containing different pathogen. The chart further includes a direct comparison with an untreated biofilm. The example cleaning mixture of Fig. 3 comprises silver particles (0.326 mg/mL) with a hydrodynamic diameter of 127 nm, manganese dioxide particles (0.001 mg/mL) with a hydrodynamic diameter of 206nm, hydrogen peroxide (1 wt.%), with the remainder of the mixture being water. The example cleaning mixture causes a 7-log reduction in the number of colony forming units (CFU) of each of the pathogens, when compared to untreated surfaces, which is well beyond the current requirements for cleaning endoscopes.

It has therefore been found that silver containing particles and manganese oxide containing particles work synergistically to very quickly remove a wide range of pathogens from the surface, such as gram-positive bacteria, gram-negative bacteria, and fungi. The cleaning mixtures described herein significantly reduce the amount of all of the tested pathogens very quickly, which is even further enhanced with the addition of hydrogen peroxide.

### Hydrodynamic diameter measurements

To obtain hydrodynamic diameter measurements of the particles described herein, dynamic light scattering may be used.

In one example, a Nanobrook 90PLUS instrument is used to obtain the dynamic light scattering measurements. In this example, to obtain the dynamic light scattering measurements of the silver particles in the silver particle stock mixture described above, the silver particle stock mixture is diluted using deionised water in the ratio 1:5 (silver particle stock mixture:deionised water). Around 2 ml of the diluted solution is then transferred by pipette into a polystyrene cuvette. The cuvette is then placed into the sample holder of the Nanobrook 90PLUS instrument and the measurement is conducted. The measurement time is 1 minute. The instrument then determines the hydrodynamic diameter. The hydrodynamic diameter is sometimes also known as the "effective diameter". An average hydrodynamic diameter can then be determined by repeating the measurement 3 times, and calculating the mean value of the 3 repeat measurements. The same procedure can be used to measure the hydrodynamic diameter of the manganese dioxide particles, using the manganese dioxide particle stock mixture rather than the silver particle stock mixture.

### Application of the cleaning mixture to a surface

Examples of the disclosure provide a method of removing biofilm from a surface, the method comprising: applying the cleaning mixtures described herein to the surface.

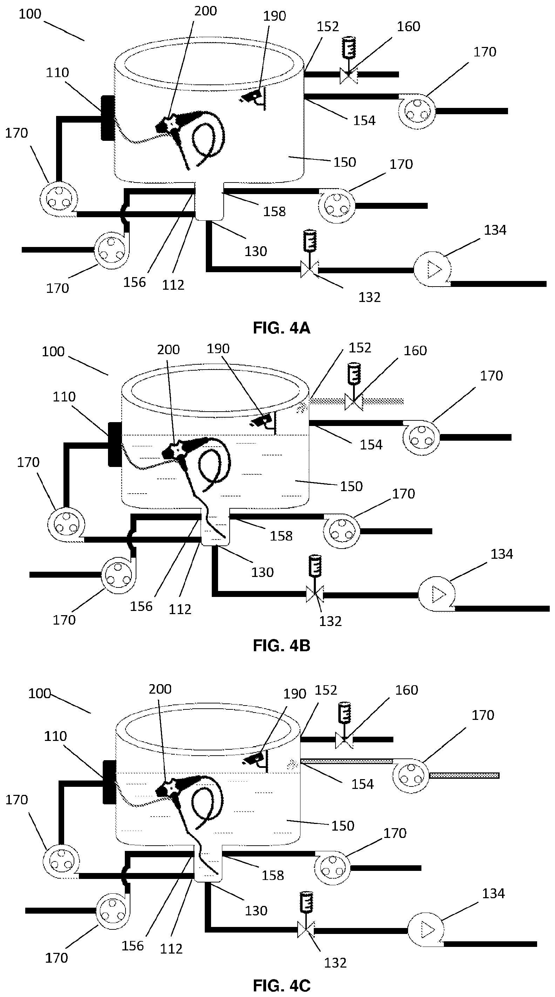

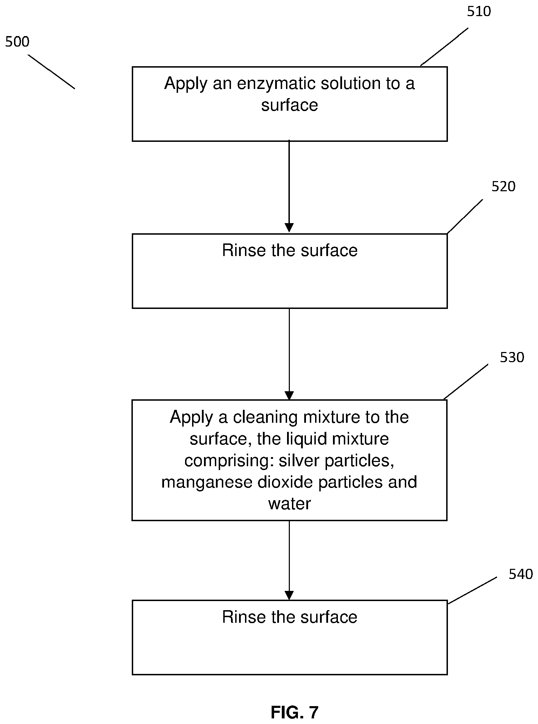

Fig. 7 shows an example method 500 for removing biofilm from a surface, and Figs. 4A to 6D illustrate a system 100 for removing biofilm from a surface. In this example, the surface is the inner surface of a tube. More particularly, the surface is the inner surface of the biopsy channel of an endoscope 200. The biopsy channel is a piece of tubing, which is arranged to allow biological samples to pass through the endoscope. The biopsy channel may be flexible.

Fig. 4A shows the system 100 prior to commencing the example method 500. The system 100 includes a reservoir (not shown) for the cleaning mixture described in the preceding sections of the description. The system further includes an inlet 110, which is arranged to direct fluid into an internal channel of the endoscope 200. The inlet 110 is also arranged to fluidly connect with the reservoir for the cleaning mixture. The internal channel of the endoscope 200 comprises a biopsy channel of the endoscope 200. In this example, the system 100 further includes a bath 150 for locating the endoscope 200, one or more inlets into the bath 150, and a drain 130 for draining liquid from the bath 150.

In the example system shown in Figs. 4A to 6D, the one or more inlets into the bath 150 includes first, second, third and fourth inlets 152, 154, 156, and 158. The first inlet 152 is a water inlet in this example, which may be fluidly connected to a mains water supply. A valve 160 is located upstream of the first inlet 152, to control the flow of water to the first inlet 152. In this example, the second inlet 154 is fluidly connected to a reservoir of enzyme solution (reservoir not shown), with a pump 170 being located upstream of the second inlet 154, to control to the flow of enzyme solution to the second inlet 154. The third inlet 156 is fluidly connected to a reservoir (not shown) of hydrogen peroxide solution. A pump 170 is located between the third inlet 156 and the reservoir of hydrogen peroxide solution, to control the flow of hydrogen peroxide solution to the third inlet 156. The fourth inlet 158 is fluidly connected to the reservoir (not shown) of the cleaning mixture, which could for instance be the first example cleaning mixture or the second example cleaning mixture described herein. Another pump 170 is located between the fourth inlet 158 and the reservoir (not shown) of the cleaning mixture to control the flow of the cleaning mixture to the fourth inlet 158. The pumps 170 could for instance be peristaltic pumps. The reservoirs described in this paragraph could for instance be plastic or glass containers containing the respective liquid.

The drain 130 is located at the bottom of the bath 150. In this example, the drain is connected to a drain valve 132, which is configured to control the draining of fluid from the bath 150. The drain 130 may also be connected to a drain pump 134, which is configured to accelerate the draining of fluid from the bath 150.

In this example, the inlet 110, which is arranged to direct fluid into the internal channel of the endoscope 200, is fluidly connected to the bath 150, via an outlet 112 from the bath 112. In this example the outlet 112 from the bath 150 is located at the bottom end of the bath 150. A pump 170, such as a peristaltic pump, may be provided between the outlet 112 from the bath 150 and the inlet 110 into the internal channel of the endoscope 200, to control the flow of liquid from the bath 150 into the endoscope 200. The endoscope 200 includes an opening, for instance at the end of the biopsy channel, to enable the liquid to flow back into the bath 150 once it has passed through the endoscope 200.

In some examples, the bath 150 further includes a sonicator 180, such as an ultrasonic sound emitter, which is configured to agitate the liquid and endoscope 200 within the bath 150. Furthermore, the bath 150 might include a level sensor 190, as shown in Figs. 2A to 4D, to detect the amount of liquid within the bath 150. When the level of liquid is too high, the level sensor 190 might trigger an alarm. Additionally, or alternatively, the level sensor 190 might be connected to a controller (not shown), which is configured to automatically carry out the method of Fig. 7 based on measurements received from the level sensor 190. The controller might be connected to the valves 132, 160 and pumps 134, 170 and be configured to control the valves 132, 160 and pumps 134, 170 to carry out the method for removing biofilm from the endoscope, such as the example method 500 of Fig. 7.

As described previously, Fig. 7 shows an example method 500 for removing biofilm from a surface, and Figs. 4A to 6D illustrate the system 100 during the steps of the method 500. The method 500 includes applying the cleaning mixture described in the preceding sections of the description to a surface. In this example, the surface is the inner surface of a tube, such as a PTFE tube. More particularly, the surface is the inner surface of the biopsy channel 210 of an endoscope 200.

In some examples, an enzymatic solution is applied to the surface prior to the application of the cleaning mixture, as shown in block 510 of Fig. 7. The enzymatic solution may be flushed through the internal channel of the endoscope 200. The enzymatic solution is a water-based solution including one or more enzymes, such as such as 3E-Zyme or Triozyme. The enzymes may be detergent enzymes to assist with breaking down the biofilm.

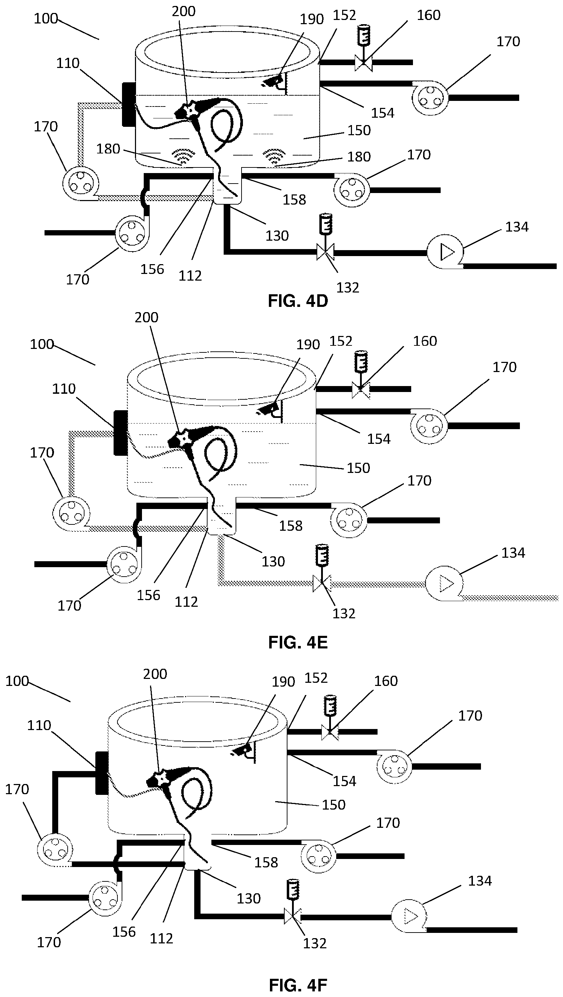

Figs. 4A to 4F illustrate an example process for applying the enzymatic solution to external and internal surfaces of an endoscope 200. Fig. 4A shows the endoscope located in the bath 150 prior to the application of the enzymatic solution. Firstly, as illustrated in Fig. 4B, water is added to the bath 150 until the endoscope 200 is immersed in water. The water is added to the bath 150, for example by opening the valve 160 connected to the first inlet 152. Enzymatic solution is then added to the bath 150. The enzymatic solution may be added to the bath 150 via the second inlet 154 by activating the pump 170 connected to the second inlet 154. Once these steps have been completed, the endoscope 200 becomes immersed in enzymatic solution, as shown in Fig. 4C. As illustrated in Fig. 4D, the sonicator 180 may then be activated to agitate the liquid and endoscope 200 within the bath 150.

In this example, the enzymatic solution is then supplied to the inlet 110 and into the internal channel of the endoscope 200. In the example of Fig. 4D, the enzymatic solution is pumped from the bath 150 to the inlet 110, via the outlet 112 from the bath 150, and then into the internal channel of the endoscope 200. Once the enzymatic solution has passed through the endoscope 200, the enzymatic solution passes out from the distal end of the endoscope 200 and returns into the bath 150. The enzymatic solution can thus circulate through the bath 150 and the internal channel of the endoscope 200 continuously.

After a period of time, the enzymatic solution is drained from the bath 150 via the drain 130, until the bath is empty, as illustrated in Figs. 4E and 4F.

In some examples, the surface with biofilm is then rinsed, as shown in block 520 of Fig. 7. The rinsing may include flushing water through the internal channel of the endoscope 200.

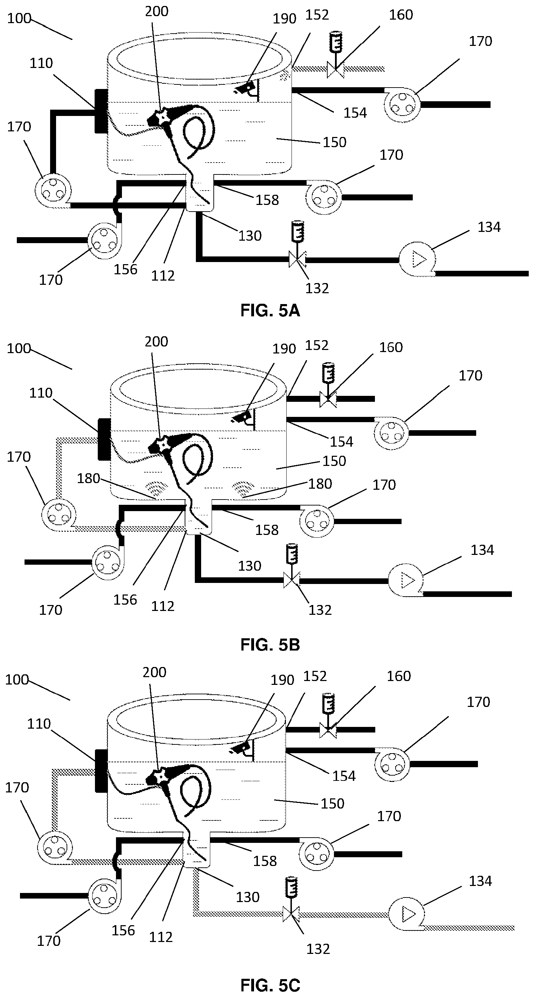

Figs. 5A to 5D illustrate an example process for rinsing the external and internal surfaces of an endoscope 200. As illustrated in Fig. 5A, water is added to the bath 150 until the endoscope 200 is immersed in water. The water is added to the bath 150 by opening the valve 160 connected to the first inlet 152. As shown in Fig. 5B, the sonicator 180 may then be activated to agitate the water and endoscope 200 within the bath 150.

To rinse the internal channel of the endoscope 200, the water is supplied to the inlet 110, which is arranged to direct the fluid into the internal channel of the endoscope 200. In the example of Fig. 5B, the water is pumped from the bath 150 to the inlet 110 via the outlet 112 from the bath 150, and into the internal channel of the endoscope 200. Once the water has passed through the endoscope 200, the water passes out from the distal end of the endoscope 200 and returns into the bath 150. Water thus can circulate through the bath 150 and the internal channel of the endoscope 200 continuously.

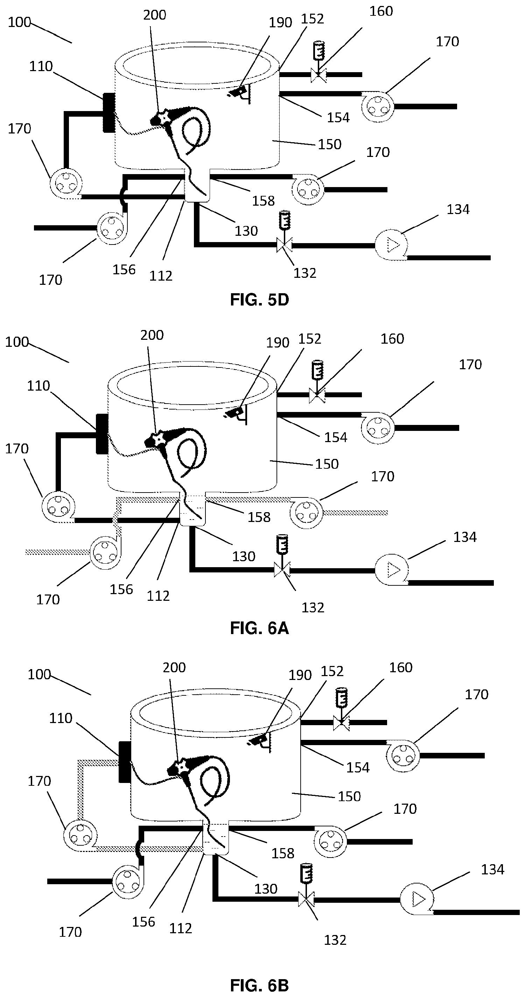

After a period of time, the water is drained from the bath 150 via the drain 130, until the bath is empty, as illustrated in Figs. 5C and 5D.

The cleaning mixture is applied to the surface, as shown in block 530 of Fig. 7. In some examples, the surface is the inner surface of a tube, and the mixture is applied by passing the mixture through the tube. In the example of Figs. 6A to 6D, the cleaning mixture is flushed through the internal channel of the endoscope 200.

In some examples, the cleaning mixture is applied to the surface for up to 15 minutes. Preferably, the cleaning mixture is applied to the surface for up to 10 minutes. Most preferably, the cleaning mixture is applied to the surface for 2.5 minutes to 7.5 minutes, such as 5 minutes.

Figs. 6A to 6D illustrate an example process for applying the cleaning mixture to the internal surfaces of an endoscope 200. As illustrated in Fig. 6A, the cleaning mixture, which could for instance be the first example cleaning mixture or the second example cleaning mixture described herein, is added to the bath 150 via the fourth inlet 158. In this example, aqueous hydrogen peroxide solution (6 wt.% solution) is also added to the bath 150 via the third inlet 156. The hydrogen peroxide solution and cleaning mixture may be pumped to the respective third and fourth inlets by the respective pumps 170. A cleaning mixture further comprising hydrogen peroxide is then formed at the bottom of the bath 150.

To remove biofilm from the internal channel of the endoscope 200, the cleaning mixture is supplied to the inlet 110 and into the internal channel of the endoscope 200. In the example of Fig. 6B, the cleaning mixture is pumped from the bath 150 to the inlet 110 via the outlet 112, and into the internal channel of the endoscope 200. Once the cleaning mixture has passed through the endoscope, the cleaning mixture passes out from the distal end of the endoscope 200 and returns into the bath 150. The cleaning mixture thus circulates through the bath 150 and the internal channel of the endoscope 200 continuously.

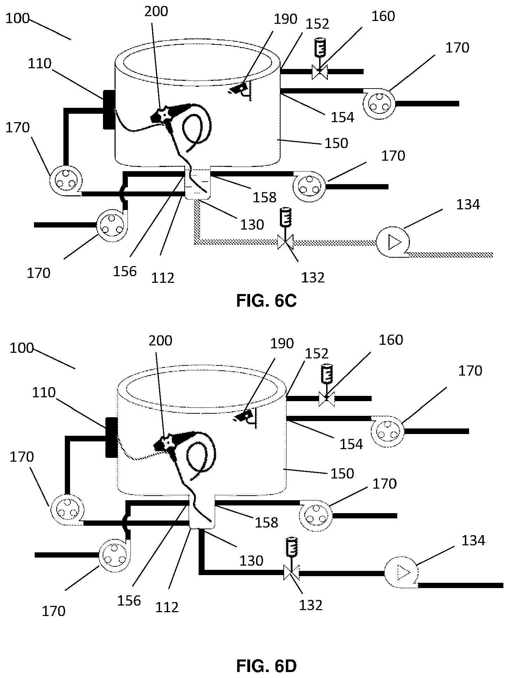

After a period of time, the cleaning mixture is drained from the bath 150 via the drain 130, until the bath is empty, as illustrated in Figs. 6C and 6D.

In some examples, the surface is then rinsed, as shown in block 540 of Fig. 7, to remove any remaining cleaning mixture from the surface. The surface could be rinsed according to the process described previously in relation to Figs. 5A to 5D.

There is thus described a cleaning mixture, a system and a method for removing biofilm from a surface with a number of advantages. The elements of the cleaning mixture work synergistically to provide a very powerful agent for removing biofilm. The biofilm can be removed very quickly, which provides a substantial advantage in hospitals where slow turnaround times can significantly increase costs. A wide spectrum of pathogens within the biofilm are also eradicated, including gram-positive bacteria, gram-negative bacteria, and fungi, which significantly reduces the infection risk from the surface. Furthermore, the mixture, system, and method do not require the use of mineral acids or other powerful acids, so biofilms can be removed from metallic surfaces without damaging or corroding the surface and surrounding elements.

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although examples have been described in the preceding paragraphs with reference to various examples, it should be appreciated that modifications to the examples given can be made without departing from the scope of the claims. For example, the cleaning mixture can be applied to a variety of surfaces, including flat, rounded, internal and/or external surfaces of a variety of objects. The mixture, method and system could be used in the food, medical, or dental industries. The cleaning mixture can be applied to the surface using a variety of methods, including for example brushing, spraying or flushing. The particles can be made using a variety of methods. The particles can be effective at a variety of sizes. Different particles for surface modification with silver or manganese oxide could be used, including other oxides such as silica particles, alumina particles or titania particles. The cleaning mixture, water, hydrogen peroxide solution and/or enzymatic solution could be added to the bath by other means. For instance, the bath might include only one inlet through which all the liquids pass into the bath.

It is to be appreciated that the silver particles could be made purely of silver, or contain some impurities such as silver oxide. Furthermore, it is to be appreciated that the manganese dioxide particles could be made purely of manganese dioxide, or contain some impurities such as manganese (III) oxide.

The manganese oxide provided on the example surface modified particles could include manganese in different oxidation states, such as oxidation states of +2 or +4. The oxidation state could depend on, for instance, the substrate and/or the particular surface site at which the manganese ion is located. Similarly, the silver provided on the surface modified particles could include silver in different oxidation states, such as oxidation states of 0 or +1.

Features described in the preceding description may be used in combinations other than the combinations explicitly described above. For instance, the cleaning mixture may include a combination of silver particles and manganese oxide surface modified particles, or a combination of manganese dioxide particles and silver surface modified particles. Where multiple ranges in the format "from X to Y" are described in relation to a certain feature, it is understood that all ranges combining the different endpoints are also contemplated.

Although functions have been described with reference to certain features, those functions may be performable by other features whether described or not.

Although features have been described with reference to certain examples, those features may also be present in other examples whether described or not.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one" or by using "consisting".

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasise an inclusive meaning but the absence of these terms should not be taken to infer any exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the applicant may seek protection via the claims in respect of any patentable feature or combination of features hereinbefore referred to and/or shown in the drawings whether or not emphasis has been placed thereon.

## Claims

1. A cleaning mixture for removing biofilm from surfaces, the mixture comprising:
silver particles and/or particles surface modified with silver;
manganese dioxide particles and/or particles surface modified with manganese oxide; and
water.

2. A mixture according to claim 1, wherein the mixture further comprises hydrogen peroxide, wherein optionally the mixture comprises up to 5 wt.% hydrogen peroxide.

3. A mixture according to claim 2, wherein the mixture comprises up to 2 wt.% hydrogen peroxide.

4. A mixture according to any of the preceding claims, wherein the mixture comprises silver particles, wherein optionally the silver particles are present in the mixture at a concentration of from 0.1 mg/mL to 1 mg/mL.

5. A mixture according to claim 4, wherein the silver particles are present in the mixture at a concentration of from 0.2 mg/mL to 0.5 mg/mL.

6. A mixture according to claim 4 or 5, wherein the silver particles have a hydrodynamic diameter of up to 1 µm, wherein optionally the silver particles have a hydrodynamic diameter of up to 200 nm.

7. A mixture according to any of the preceding claims, wherein the mixture comprises manganese dioxide particles, wherein optionally the manganese dioxide particles are present in the mixture at a concentration of from 0.0002 mg/mL to 0.005 mg/mL.

8. A mixture according to claim 7, wherein the manganese dioxide particles are present in the mixture at a concentration of from 0.0005 mg/mL to 0.003 mg/mL.

9. A mixture according to claim 7 or 8, wherein the manganese dioxide particles have a hydrodynamic diameter of up to 1 µm, wherein optionally the manganese dioxide particles have a hydrodynamic diameter of up to 500 nm.

10. A mixture according to any of claims 4 to 9, wherein the mixture further comprises a capping agent, wherein optionally the capping agent is a water-soluble polymer capping agent, wherein optionally the polymer capping agent is polyvinylpyrrolidone.

11. A mixture according to any of the preceding claims, wherein the mixture comprises silver particles and manganese dioxide particles.

12. A mixture according to any of the preceding claims, wherein the mixture comprises particles surface modified with silver and particles surface modified with manganese oxide, wherein optionally at least some of the particles surface modified with silver and at least some of the particles surface modified with manganese oxide are particles surface modified with both silver and manganese oxide.

13. A method (500) of removing biofilm from a surface, the method comprising:
applying the cleaning mixture of any of the preceding claims to the surface.

14. A method according to claim 13 when dependent on claim 2, wherein the hydrogen peroxide is added to the mixture up to 5 minutes before applying the mixture to the surface.

15. A system (100) for removing biofilm from the biopsy channel of an endoscope (200), the system comprising:
a reservoir containing the cleaning mixture of any of claims 1 to 12; and
an inlet (110), which is arranged to direct fluid into an internal channel of the endoscope.

## Patentansprüche

1. Reinigungsgemisch zum Entfernen eines Biofilms von Oberflächen, wobei das Gemisch Folgendes umfasst:
Silberpartikel und/oder Partikel, die mit Silber oberflächenmodifiziert sind;
Mangandioxidpartikel und/oder Partikel, die mit Manganoxid oberflächenmodifiziert sind; und
Wasser.

2. Gemisch nach Anspruch 1, wobei das Gemisch ferner Wasserstoffperoxid umfasst, wobei das Gemisch optional bis zu 5 Gew.-% Wasserstoffperoxid umfasst.

3. Gemisch nach Anspruch 2, wobei das Gemisch bis zu 2 Gew.-% Wasserstoffperoxid umfasst.

4. Gemisch nach einem der vorhergehenden Ansprüche, wobei das Gemisch Silberpartikel umfasst, wobei die Silberpartikel optional in dem Gemisch in einer Konzentration von 0,1 mg/ml bis 1 mg/ml vorliegen.

5. Gemisch nach Anspruch 4, wobei die Silberpartikel in dem Gemisch in einer Konzentration von 0,2 mg/ml bis 0,5 mg/ml vorliegen.

6. Gemisch nach Anspruch 4 oder 5, wobei die Silberpartikel einen hydrodynamischen Durchmesser von bis zu 1 µm aufweisen, wobei die Silberpartikel optional einen hydrodynamischen Durchmesser von bis zu 200 nm aufweisen.

7. Gemisch nach einem der vorhergehenden Ansprüche, wobei das Gemisch Mangandioxidpartikel umfasst, wobei die Mangandioxidpartikel optional in dem Gemisch in einer Konzentration von 0,0002 mg/ml bis 0,005 mg/ml vorliegen.

8. Gemisch nach Anspruch 7, wobei die Mangandioxidpartikel in dem Gemisch in einer Konzentration von 0,0005 mg/ml bis 0,003 mg/ml vorliegen.

9. Gemisch nach Anspruch 7 oder 8, wobei die Mangandioxidpartikel einen hydrodynamischen Durchmesser von bis zu 1 µm aufweisen, wobei die Mangandioxidpartikel optional einen hydrodynamischen Durchmesser von bis zu 500 nm aufweisen.

10. Gemisch nach einem der Ansprüche 4 bis 9, wobei das Gemisch ferner ein Verkappungsmittel umfasst, wobei das Verkappungsmittel optional ein wasserlösliches Polymerverkappungsmittel ist, wobei das Polymerverkappungsmittel optional Polyvinylpyrrolidon ist.

11. Gemisch nach einem der vorhergehenden Ansprüche, wobei das Gemisch Silberpartikel und Mangandioxidpartikel umfasst.

12. Gemisch nach einem der vorhergehenden Ansprüche, wobei das Gemisch Partikel, die mit Silber oberflächenmodifiziert sind, und Partikel, die mit Manganoxid oberflächenmodifiziert sind, umfasst, wobei optional mindestens einige der Partikel, die mit Silber oberflächenmodifiziert sind, und mindestens einige der Partikel, die mit Manganoxid oberflächenmodifiziert sind, Partikel sind, die sowohl mit Silber als auch mit Manganoxid oberflächenmodifiziert sind.

13. Verfahren (500) zum Entfernen eines Biofilms von einer Oberfläche, wobei das Verfahren Folgendes umfasst:
Auftragen des Reinigungsgemisches nach einem der vorhergehenden Ansprüche auf die Oberfläche.

14. Verfahren nach Anspruch 13 in Abhängigkeit von Anspruch 2, wobei das Wasserstoffperoxid dem Gemisch bis zu 5 Minuten vor dem Aufbringen des Gemisches auf die Oberfläche zugegeben wird.

15. System (100) zum Entfernen eines Biofilms aus dem Biopsiekanal eines Endoskops (200), wobei das System Folgendes umfasst:
einen Behälter, der das Reinigungsgemisch nach einem der Ansprüche 1 bis 12 enthält; und
einen Einlass (110), der angeordnet ist, um Fluid in einen inneren Kanal des Endoskops zu leiten.

## Revendications

1. Mélange de nettoyage destiné à éliminer le biofilm de surfaces, le mélange comprenant :
des particules d'argent et/ou des particules modifiées en surface avec de l'argent ;
des particules de dioxyde de manganèse et/ou des particules modifiées en surface avec de l'oxyde de manganèse ; et
de l'eau.

2. Mélange selon la revendication 1, ledit mélange comprenant en outre du peroxyde d'hydrogène, ledit mélange comprenant éventuellement jusqu'à 5 % en poids de peroxyde d'hydrogène.

3. Mélange selon la revendication 2, ledit mélange comprenant jusqu'à 2 % en poids de peroxyde d'hydrogène.

4. Mélange selon l'une quelconque des revendications précédentes, ledit mélange comprenant des particules d'argent, lesdites particules d'argent étant éventuellement présentes dans ledit mélange à une concentration allant de 0,1 mg/mL à 1 mg/mL.

5. Mélange selon la revendication 4, lesdites particules d'argent étant présentes dans le mélange à une concentration allant de 0,2 mg/mL à 0,5 mg/mL.

6. Mélange selon la revendication 4 ou 5, lesdites particules d'argent possédant un diamètre hydrodynamique allant jusqu'à 1 µm, lesdites particules d'argent possédant éventuellement un diamètre hydrodynamique allant jusqu'à 200 nm.

7. Mélange selon l'une quelconque des revendications précédentes, ledit mélange comprenant des particules de dioxyde de manganèse, lesdites particules de dioxyde de manganèse étant éventuellement présentes dans le mélange à une concentration allant de 0,0002 mg/mL à 0,005 mg/mL.

8. Mélange selon la revendication 7, lesdites particules de dioxyde de manganèse étant présentes dans le mélange à une concentration allant de 0,0005 mg/mL à 0,003 mg/mL.

9. Mélange selon la revendication 7 ou 8, lesdites particules de dioxyde de manganèse possédant un diamètre hydrodynamique allant jusqu'à 1 µm, lesdites particules de dioxyde de manganèse possédant éventuellement un diamètre hydrodynamique allant jusqu'à 500 nm.

10. Mélange selon l'une quelconque des revendications 4 à 9, ledit mélange comprenant en outre un agent de coiffage, ledit agent de coiffage étant éventuellement un agent de coiffage polymère hydrosoluble, ledit agent de coiffage polymère étant éventuellement de la polyvinylpyrrolidone.

11. Mélange selon l'une quelconque des revendications précédentes, ledit mélange comprenant des particules d'argent et des particules de dioxyde de manganèse.

12. Mélange selon l'une quelconque des revendications précédentes, ledit mélange comprenant des particules modifiées en surface avec de l'argent et des particules modifiées en surface avec de l'oxyde de manganèse, au moins une partie des particules modifiées en surface avec de l'argent et au moins une partie des particules modifiées en surface avec de l'oxyde de manganèse étant éventuellement des particules modifiées en surface avec, à la fois, de l'argent et de l'oxyde de manganèse.

13. Procédé (500) d'élimination d'un biofilm d'une surface, le procédé comprenant :
l'application du mélange de nettoyage de l'une quelconque des revendications précédentes à la surface.

14. Procédé selon la revendication 13 lorsqu'elle dépend de la revendication 2, ledit peroxyde d'hydrogène étant ajouté au mélange jusqu'à 5 minutes avant d'appliquer le mélange sur la surface.

15. Système (100) destiné à éliminer un biofilm du canal de biopsie d'un endoscope (200), le système comprenant :
un réservoir contenant le mélange de nettoyage de l'une quelconque des revendications 1 à 12 ; et
une entrée (110), qui est agencée pour diriger le fluide dans un canal interne de l'endoscope.
